Europäisches Patentamt

European Patent Office

Office européen des brevets

(19)

(11) Veröffentlichungsnummer: **0 386 714**
**A2**

(12) **EUROPÄISCHE PATENTANMELDUNG**

(21) Anmeldenummer: 90104309.1

(51) Int. Cl.⁵: **A61L 9/01**

(22) Anmeldetag: **07.03.90**

(30) Priorität: 09.03.89 DE 3907555

(43) Veröffentlichungstag der Anmeldung:
**12.09.90 Patentblatt 90/37**

(84) Benannte Vertragsstaaten:
**AT CH DE ES FR GB IT LI**

(71) Anmelder: **HOECHST AKTIENGESELLSCHAFT**
**Postfach 80 03 20**
**D-6230 Frankfurt am Main 80(DE)**

(72) Erfinder: **Gscheidmeier, Manfred, Dr.**
**Goethestrasse 4**
**D-8901 Gablingen(DE)**
Erfinder: **Bender, Philipp**
**Nachtigallenweg 9a**
**D-6238 Hofheim am Taunus(DE)**

(54) **Verwendung von Monoterpenen als Luftverbesserer.**

(57) Die Monoterpene Borneol, Isoborneol, Kampfer und Isobornylacetat eignen sich auch als Reinsubstanz als Luftverbesserer, auch im Bestattungswesen.

EP 0 386 714 A2

# Verwendung von Monoterpenen als Luftverbesserer

Die Erfindung bezieht sich auf die Verwendung von Monoterpenen als Luftverbesserer und Insektenrepellent im Bestattungswesen und im Sanitärbereich.

Luftverbesserer (Desodorierungsmittel) sollen unangenehme Gerüche binden oder überdecken. Vorteilhaft sollen sie gleichzeitig als Insektenrepellent, besonders gegen Fliegen und Mücken, wirken.

Sowohl im Sanitärbereich als auch im Bestattungswesen treten üblicherweise Gerüche auf, die für dort Beschäftigte, besonders in der warmen Jahreszeit, außerordentlich unangenehm sein können. Die gleichzeitig auftretenden Insekten bilden eine Plage und bringen Gesundheitsrisiken mit sich.

Als Gegenmittel werden Desodorierungsmittel eingesetzt, die bisher im Sanitärbereich weitgehend und im Bestattungswesen ausschließlich p-Dichlorbenzol enthielten.

Dies ist in toxikologischer und in ökologischer Hinsicht nicht mehr vertretbar. Selbst wenn p-Dichlorbenzol nur als mindergiftig eingestuft ist, muß man, wie bei allen chlorierten Aromaten, davon ausgehen, daß Folgeprodukte hiervon für den Menschen und für andere Lebewesen toxisch wirken und bei Verbrennungsvorgängen zu gefährlichen Abbauprodukten, vermutlich sogar zu den als sehr giftig einzustufenden Dioxinen führen können.

Außerdem hält sich die geruchsverbessernde Wirkung in Grenzen, da p-Dichlorbenzol selbst sehr aufdringlich und unfein riecht.

Es ist ein Desodorierungsmittel bekannt, welches vorzugsweise als Luftreinigungsmittel und in Toiletteneinrichtungen verwendet wird und welches eine "Kampferverbindung" umfaßt (vgl. WO 85/00980). Die "Kampferverbindung" ist Borneol, Isoborneol, Camphen oder Kampfer und wird in einer Menge von 1 bis 50 Gew.-% eingesetzt. Daneben enthält das Desodorierungsmittel Detergentien, Polywachse und Füllstoffe.

Weiterhin ist die Eignung von reinem Isoborneol als Kleidermotten abweisendes Mittel bekannt (vgl. EP 285983).

Es wurde nun gefunden, daß die gewünschte Wirkung als Luftverbesserer und Insektenrepellent gut von einem Monoterpenderivat allein erreicht wird. Verwendet werden Borneol, Isoborneol, Kampfer oder Isobornylacetat, vorzugsweise Isoborneol, wobei diese Monoterpene keine weiteren Zusatzstoffe benötigen.

In toxikologischer und ökologischer Hinsicht sind diese Monoterpene unbedenklich, wie folgende Angaben zeigen: ·

$LD_{50}$ (Ratte, oral): Kampfer über 5000 mg/kg

Borneol 5800-7200 mg/kg
Isoborneol 4300-6200 mg/kg
Isobornylacetat über 10000 mg/kg

Im Ames-Test zeigen Kampfer, Isoborneol und Isobornylacetat kein mutagenes Potential.

In gesättigter Lösung weisen sie eine Abbaubarkeit von ca. 90 % in 5 Tagen auf.

Beim Verbrennen unter ausreichender Sauerstoffzufuhr ist mit der Bildung von gefährlichen Susbtanzen nicht zu rechnen.

Die Anwendung von Borneol, Isoborneol, Kampfer und Isobornylacetat als Luftverbesserer im Bestattungswesen oder Sanitär-Deodorant ist allein oder in Abmischung in folgenden Konfektionierungsformen möglich:
als grob- oder feingemahlenes Pulver,
als grobkörniges Granulat,
als Gieß- oder Preßkörper,
als Flüssigkeit oder in Form einer Lösung (z.B. in Ethanol), wobei das rasch verdunstende Lösemittel nur zur gleichmäßigen und guten Oberflächenverteilung, nicht aber als Wirkstoff dient.

Während Gieß- oder Presskörper vorzugsweise die Umgebungsluft verbessern und Insekten anhaltend abweisen sollen, wird das streufähige Material zur intensiveren Wirkung auf eine größere Fläche verteilt. Besser ist jedoch die Wirkung beim Versprühen, Vergießen oder Pinselauftrag einer Lösung, z.B. von 55 % Isoborneol in 25 % Isobornylacetat und 20 % Ethanol, da nach dem Antrocknen (Verdampfen des Lösemittels) ein weicher filmartiger Belag mit stark geruchsbindender Wirkung zurückbleibt.

Sollte eine geruchliche Variation erreicht werden, so ist der Zusatz von Riechstoffen möglich.

## Ansprüche

1. Verwendung der Monoterpene Borneol, Isoborneol, Kampfer und Isobornylacetat als Luftverbesserer im Bestattungswesen.

2. Verwendung nach Anspruch 1, dadurch gekennzeichnet, daß das Monoterpen als Reinsubstanz eingesetzt wird.

3. Verwendung nach Anspruch 1, dadurch gekennzeichnet, daß das Monoterpen Isoborneol ist.

4. Verwendung nach Anspruch 1, dadurch gekennzeichnet, daß das Isoborneol in Form einer Lösung eingesetzt wird.